# EUROPÄISCHE PATENTSCHRIFT

(11) **EP 0 837 911 B1**
(45) Veröffentlichungstag und Bekanntmachung des Hinweises auf die Patenterteilung: **17.10.2001**
(21) Anmeldenummer: 97921766.8
(22) Anmeldetag: 25.04.1997
(51) Int. Cl.: C09C 1/00, C09C 1/36, C09D 7/12, C09D 11/00, C08K 3/00, A61K 7/00, C04B 33/14, A61K 7/42, A23L 1/275

(54) **PLÄTTCHENFÖRMIGES TITANDIOXIDPIGMENT**
PLATE-LIKE TITANIUM DIOXIDE PIGMENT
PIGMENT DE DIOXYDE DE TITANE SOUS FORME DE PLAQUETTES

(30) Priorität: 09.05.1996 DE 19618564
(43) Veröffentlichungstag der Anmeldung: 29.04.1998
(73) Patentinhaber: MERCK PATENT GmbH, 64293 Darmstadt (DE)
(72) Erfinder: ANDES, Stephanie, D-63477 Maintal 3 (DE); BAUER, Gerd, D-63801 Kleinostheim (DE); BRENNER, Günter, D-64347 Griesheim (DE); BRÜCKNER, Dieter, D-64289 Darmstadt (DE); HEYLAND, Andrea, D-64385 Ober-Kainsbach (DE); KUNTZ, Matthias, D-64342 Seeheim (DE); OSTERRIED, Karl, D-64807 Dieburg (DE); PFAFF, Gerhard, D-64839 Münster (DE); SCHMELZ, Michael, D-65830 Kriftel (DE)
(86) Internationale Anmeldenummer: EP9702132
(87) Internationale Veröffentlichungsnummer: WO9743346

(56) Entgegenhaltungen:
- EP-A- 0 649 816
- US-A- 3 340 006
- US-A- 3 395 203
- US-A- 3 582 382
- DATABASE WPI Week 8948 Derwent Publications Ltd., London, GB; AN 89-353145 XP002035808 & JP 01 264 932 A (ISHIHARA SANGYO KAISHA) , 23.Oktober 1989

## Beschreibung

Die Erfindung betrifft ein sehr dünnes Titandioxid-Perlglanzpigment auf der Basis von plättchenförmigem Titandioxid.

Titandioxidhaltige Perlglanzpigmente werden seit vielen Jahren mit Erfolg angewendet. Sie sind nach dem Schichtsubstrat-Prinzip aufgebaut, wobei als Substrat fast ausnahmslos Glimmer eingesetzt wird.

Für die Auffällung der TiO₂-Schicht sind im wesentlichen 2 Verfahren bekannt. So kann die Fällung, wie z.B. im US-Patent 30 87 828 beschrieben, durch Zugabe einer schwefelsauren Titanylsulfatlösung zur Glimmersuspension und deren Hydrolyse durch Erhitzen auf etwa 100 °C erfolgen, wobei die Schichtdicke und die damit verbundene Interferenzfarbe von vornherein durch die Menge des anwesenden Titanylsulfats vorgegeben ist.

Die Fällung kann aber auch so wie beispielsweise in der deutschen Patentschrift 20 09 566 beschrieben, durchgeführt werden. Dabei wird zu einer auf etwa 50-100 °C, insbesondere 70-80 °C erhitzten Glimmersuspension langsam eine wäßrige Titansalzlösung zugegeben, und es wird durch gleichzeitiges Zudosieren einer Base, wie z.B. wäßrige Ammoniaklösung oder eine wäßrige Alkalilauge, ein weitgehend konstanter pH-Wert von etwa 0,5-5, insbesondere etwa 1,5-2,5 eingehalten. Sobald die gewünschte Schichtdicke der TiO₂-Fällung erreicht ist, wird die Zugabe der Titansalzlösung gestoppt.

Glimmer hat den Nachteil, daß er bei der Titandioxidbildung die Anatasmodifikation induziert, obwohl häufig die Rutilmodifikation erwünscht ist, die eine höhere Brechzahl und noch weitere vorteilhafte Eigenschaften besitzt. Die Rutilmodifikation muß deshalb durch Zusatz von Fremdionen, insbesondere Zinn(IV)-ionen, erzwungen werden.

Derartige Verfahren sind z.B. in den deutschen Patentschriften 22 14 545 und 25 22 572 beschrieben, wobei durch Einbau von Zinndioxid in Glimmernähe oder in diskreten Schichten zwischen dem TiO₂ die Rutilisierung erzwungen wird. Es sind aber auch andere Verfahren bekannt, wie z.B. der Einbau von Zinkoxid entsprechend der CS-PS 208,578 oder der Einbau von Eisen(III) in die TiO₂-Schicht entsprechend der DE-PS 19 59 998, die zu Rutilschichten führen.

Glimmerpigmente werden in großem Umfang in der Druck- und Lackindustrie, in der Kosmetik und in der Kunststoffverarbeitung verwendet. Sie zeichnen sich durch Interferenzfarben und einen hohen Glanz aus. Für die Ausbildung extrem dünner Schichten sind aber Glimmerpigmente nicht geeignet, weil Glimmer als Substrat für die Metalloxidschichten des Pigmentes bereits eine Dicke von 200 bis 1200 nm aufweist. Nachteilig ist weiterhin, daß die Dicke der Glimmerplättchen innerhalb einer bestimmten Fraktion, die durch die Plättchengröße bestimmt wird, teilweise deutlich um einen Mittelwert schwankt. Außerdem handelt es sich bei Glimmer um ein natürlich vorkommendes Mineral, das durch Fremdionen verunreinigt ist. Desweiteren sind technisch sehr aufwendige und zeitraubende Aufbereitungsschritte notwendig. Hierzu zählen vor allem Mahlen und Klassieren.

Auf dicken Glimmerplättchen basierende, mit Metalloxiden umhüllte Perlglanzpigmente haben aufgrund der Dicke des Randes einen deutlichen Streuanteil, speziell bei feineren Korngrößenverteilungen unter 20 µm.

Als Ersatz für Glimmer sind dünne Glasplättchen vorgeschlagen worden, die durch Walzen einer Glasschmelze mit nachfolgendem Mahlen erhalten werden. Interferenzpigmente auf der Basis derartiger Materialien weisen zwar Farbeffekte auf, die denen herkömmlicher, auf Glimmer basierender Pigmente überlegen sind. Nachteilig ist jedoch, daß die Glasplättchen eine sehr große mittlere Dicke von etwa 10-15 µm und eine sehr breite Dickenverteilung (typischerweise zwischen 4 und 20 µm) aufweisen, während die Dicke von Interferenzpigmenten typischerweise nicht größer als 3 µm ist.

In EP 0,384,596 wird ein Verfahren beschrieben, bei dem hydratisiertes Alkalisilikat bei Temperaturen von 480-500 °C mit einem Luftstrahl beaufschlagt wird, wobei sich Blasen mit dünnen Wandstärken bilden; die Blasen werden anschließend zerkleinert und man erhält plättchenförmige Alkalisilikatsubstrate mit einer Dicke von weniger als 3 µm. Das Verfahren ist jedoch aufwendig und die Dickenverteilung der erhaltenen Plättchen ist relativ breit.

In DE 11 36 042 ist ein kontinuierliches Bandverfahren zur Herstellung plättchen- oder flitterartiger Oxide oder Oxidhydrate von Metallen der IV. und V. Gruppe sowie der Eisen-Gruppe des Periodensystems beschrieben. Dabei wird auf ein kontinuierliches Band ggf. zunächst eine Trennschicht aus z.B. Silikonlack aufgebracht, um das spätere Ablösen der Metalloxidschicht zu erleichtern. Anschließend wird ein Flüssigkeitsfilm aus einer Lösung einer hydrolysierbaren Verbindung des in das gewünschte Oxid umzuwandelnden Metalls aufgebracht, der Film wird getrocknet und anschließend mit einer Rüttelvorrichtung abgelöst. Die Schichtdicke der erhaltenen Plättchen wird mit 0,2 bis 2 µm angegeben, ohne hierfür konkrete Beispiele zu nennen.

In EP 0,240,952 und EP 0,236,952 ist ein kontinuierliches Bandverfahren zur Herstellung verschiedener plättchenförmiger Materialien, darunter auch Siliciumdioxid, Aluminiumoxid und Titandioxid vorgeschlagen worden. Dabei wird über ein Rollensystem auf ein glattes Band ein dünner flüssiger Film definierter Dickes eines Precursors des plättchenförmigen Materials aufgebracht; der Film wird getrocknet und von dem Band abgelöst, wobei sich plättchenförmige Teilchen bilden. Die Teilchen werden anschließend gegebenenfalls geglüht, gemahlen und klassiert.

Die Dicke der nach dem in EP 0 240 952 beschriebenen Verfahren erhaltenen Plättchen ist relativ gut definiert, da der Film z.B. auf das kontinuierliche Band über ein Rollensystem sehr gleichmäßig aufgebracht wird. Die Schichtdicke der Plättchen wird in den Beispielen mit 0,3 bis 3,0 µm angegeben. Gemäß Beispiel 1 wird eine erste Rolle mit dem verwendeten Precursor benetzt, indem man diese Rolle teilweise in einen mit dem Precursor befüllten Vorratsbehälter eintaucht. Der Film wird von dieser Rolle auf eine zweite, gleichsinnig rotierende Rolle übertragen, die mit der ersten in sehr engem Kontakt steht. Schließlich wird der Film von der zweiten Rolle auf das kontinuierliche Band abgerollt.

Nachteilig sind jedoch die Verwendung sehr teurer Precursormaterialien sowie insbesondere die erhöhten Anforderungen an die Arbeitsplatzsicherheit, die beim Einsatz metallorganischer Verbindungen gestellt werden müssen. Die vollständige chemische Umwandlung des Precursors in das gewünschte Schichtmaterial macht in der Regel eine starke Erhitzung des Filmes und des Bandmaterials erforderlich. Neben der dabei auftretenden erheblichen thermischen Belastung des Bandmaterials, wirken sich auch der hohe Energieaufwand und die Einschränkung der Prozeßgeschwindigkeit sehr nachteilig auf die Wirtschaftlichkeit des Verfahrens aus.

WO 93/08 237 beschreibt plättchenförmige Pigmente, bestehend aus einer plättchenförmigen Matrix aus Siliciumdioxid, die lösliche oder nichtlösliche Farbmittel enthalten kann und die mit einer oder mehreren reflektierenden Schichten aus Metalloxiden oder Metallen belegt ist. Die plättchenförmige Matrix wird durch Verfestigung von Wasserglas auf einem endlosen Band hergestellt.

In DE 1 273 098 wird die Herstellung eines Perlmuttpigmentes durch Aufdampfen von ZnS-, MgF₂-, ZnO-, CaF₂- und TiO₂-Filmen auf ein endloses Band beschrieben. Dieses Verfahren ist aber ebenso, wie das in US 4 879 140 beschriebene Verfahren, bei dem plättchenförmige Pigmente mit Si- und SiO₂-Schichten durch Plasmaabscheidung aus SiH₄ und SiCl₄ erhalten werden, mit einem sehr hohen apparativen Aufwand verbunden.

Trotz zahlreicher Versuche konnte bisher noch kein wirtschaftliches Verfahren zur Herstellung sehr dünner plättchenförmiger Titandioxidpigmente mit einer Schichtdicke von kleiner als 500 nm entwickelt werden.

Aufgabe der Erfindung ist es, hochglänzendes Perlglanzpigment aus Titandioxid mit einer Schichtdicke von kleiner als 500 nm und einer Schichtdickentoleranz von weniger als 10 % bereitzustellen, wobei das Pigment praktisch keine Fremdionen enthält und in der Rutil- oder der Anatasform vorliegt.

Diese Aufgabe wird gemäß der Erfindung gelöst durch ein plättchenförmiges Titandioxidpigment, erhältlich durch Verfestigung einer wäßrigen Lösung einer thermisch hydrolysierbaren Titanverbindung auf einem endlosen Band, Ablösung der entstandenen Schicht, Beschichtung der erhaltenen Titandioxidplättchen ohne Zwischentrocknung im Naßverfahren mit weiterem Titandioxid, Abtrennung, Trocknung und Kalzination des erhaltenen Materials.

Als thermisch hydrolysierbare Titanverbindung wird bevorzugt eine wäßrige Titantetrachloridlösung verwendet. Die Konzentration des Titansalzes in diesen Lösungen beträgt 7 bis 30 Gew.-%, vorzugsweise 8 bis 15 Gew.-%.

Weiterhin wird diese Aufgabe gemäß der Erfindung gelöst durch ein Verfahren zur Herstellung des erfindungsgemäßen Pigmentes, indem
- ein Precursor des plättchenförmigen Titandioxides als dünner Film auf ein endloses Band aufgebracht wird,
- der flüssige Film durch Trocknung verfestigt wird und dabei das Titandioxid durch eine chemische Reaktion aus dem Precursor entwickelt wird,
- die entstandene Schicht anschließend vom Band abgelöst und gewaschen wird,
- die erhaltenen Titandioxidplättchen ohne Zwischentrocknung in Wasser suspendiert und mit weiterem Titandioxid beschichtet werden,
- die beschichteten Titandioxidplättchen aus der wäßrigen Suspension abgetrennt, getrocknet und gegebenenfalls kalziniert werden.

Das Titandioxid liegt nach dem Trocknen in der Anatasmodifikation vor. Durch Kalzinieren oberhalb von 600 °C kann es ohne Anwesenheit von Fremdionen in die Rutilform überführt werden. Dadurch wird ein sehr reines Titandioxidpigment in der Rutilform erhalten, das den herkömmlichen Titandioxidpigmenten auf Glimmerbasis in vielen Belangen überlegen ist.

Wird Zinn in die TiO₂-Matrix eingebaut, erfolgt bereits beim Trocknen ab 110 °C eine Umwandlung in die Rutilform.

Gegenstand der Erfindung ist außerdem die Verwendung des erfindungsgemäßen Pigmentes zur Pigmentierung von Lacken, Druckfarben, Kunststoffen, Kosmetika und Glasuren für Keramiken und Gläser.

Hierfür können sie auch als Mischungen mit handelsüblichen Pigmenten, beispielsweise anorganischen und organischen Absorptionspigmenten, Metalleffektpigmenten und LCP-Pigmenten, eingesetzt werden.

Das erfindungsgemäße Pigment besteht aus plättchenförmigem Titandioxid. Diese Plättchen haben eine Dicke zwischen 10 nm und 500 nm, vorzugsweise zwischen 40 und 300 nm. Die Ausdehnung in die beiden anderen Dimensionen beträgt zwischen 2 und 200 µm und insbesondere zwischen 5 und 50 µm.

Die Herstellung des erfindungsgemäßen Pigmentes erfolgt in einem zweistufigen Verfahren. In der ersten Stufe werden Titandioxidplättchen mit Hilfe eines endlosen Bandes hergestellt.

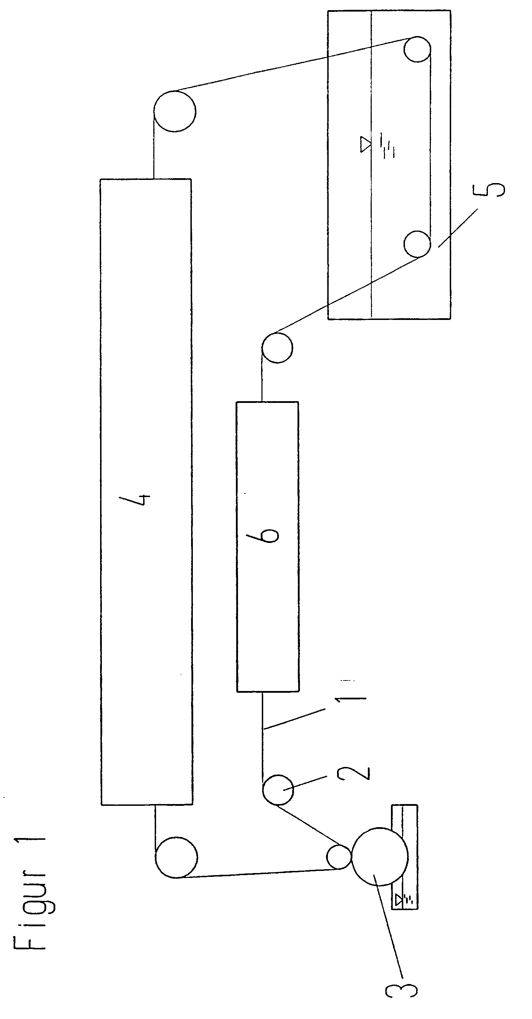

Zunächst soll an Hand von Figur 1 das Bandverfahren erläutert werden.

Das endlose Band 1, welches über ein Rollensystem 2 geführt wird, durchläuft ein Auftragswerk 3, wo es mit einem dünnen Film des Precursors beschichtet wird. Als geeignete Auftragswerke können Walzenauftragswerke sowie Fließer eingesetzt werden. Die Bandgeschwindigkeit liegt zwischen 2 und 400 m/min, vorzugsweise 5-200 m/min.

Um eine gleichmäßige Benetzung des Kunststoffbandes zu erzielen, ist es zweckmäßig, der Beschichtungslösung ein handelsübliches Netzmittel zuzusetzen oder die Bandoberfläche durch Beflammung, Koronabehandlung oder lonisation zu aktivieren.

Anschließend durchläuft das beschichtete Band eine Trockenstrecke 4 in der die Schicht bei Temperaturen zwischen 30 und 200 °C getrocknet wird. Als Trockner können zum Beispiel handelsübliche Infrarot-, Umluftdüsen- und UV-Trockner eingesetzt werden.

Nach dem Passieren der Trockenstrecke wird das Band durch die Ablösebäder 5 mit einem geeignetem Ablösemedium, zum Beispiel vollentsalztem Wasser geführt, wo die getrocknete Schicht vom Band entfernt wird. Der Ablösevorgang wird hierbei durch zusätzliche Vorrichtungen, zum Beispiel Düsen, Bürsten oder Ultraschall, unterstützt.

In einem Nachtrockner 6 wird das Band vor der erneuten Beschichtung getrocknet.

Das endlose Band sollte aus einem chemisch und thermisch beständigem Kunststoff sein, um eine ausreichende Standzeit und hohe Trocknungstemperaturen zu gewährleisten. Hierfür sind Materialien wie Polyethylenterephthalat (PET) oder andere Polyester und Polyacrylate geeignet.

Die Folienbreite liegt typischerweise zwischen einigen Zentimetern bis zu mehreren Metern. Die Dicke beträgt zwischen 10 µm bis zu einigen mm, wobei diese beiden Parameter im Hinblick auf die jeweiligen Anforderungen optimiert werden.

Weitere Einzelheiten zu kontinuierlichen Bandverfahren sind aus US 3 138 475, EP 0 240 952 und WO 93/08 237 bekannt.

Die vom Band abgelösten Titandioxidplättchen werden in einer zweiten Verfahrensstufe ohne vorherige Zwischentrocknung mit weiterem Titandioxid nach bekanntem Verfahren beschichtet. Bevorzugt wird das in US 3 553 001 beschriebene Verfahren verwendet.

Zu einer auf etwa 50-100 °C, insbesondere 70-80 °C erhitzten Suspension der Titandioxidplättchen wird langsam eine wäßrige Titansalzlösung zugegeben, und es wird durch gleichzeitiges Zudosieren einer Base, wie z.B. wäßrige Ammoniaklösung oder eine wäßrige Alkalilauge, ein weitgehend konstanter pH-Wert von etwa 0,5-5, insbesondere etwa 1,5-2,5 eingehalten. Sobald die gewünschte Schichtdicke der TiO₂-Fällung erreicht ist, wird die Zugabe der Titansalzlösung gestoppt.

Dieses, auch als Titrationsverfahren bezeichnete Verfahren zeichnet sich dadurch aus, daß ein Überschuß an Titansalz vermieden wird. Das wird dadurch erreicht, daß man pro Zeiteinheit nur eine solche Menge der Hydrolyse zuführt, wie sie für eine gleichmäßige Beschichtung mit dem hydratisierten TiO₂ erforderlich ist und wie pro Zeiteinheit von der verfügbaren Oberfläche der zu beschichtenden Teilchen aufgenommen werden kann. Es entstehen deshalb fast keine hydratisierten Titandioxidteilchen, die nicht auf der zu beschichtenden Oberfläche niedergeschlagen sind. Die pro Minute zugesetzte Titansalzmenge liegt dabei in der Größenordnung von etwa 0,01 bis 2 · 10⁻⁴ Mol Titansalz pro Quadratmeter zu belegender Oberfläche.

Weiterhin kann die Beschichtung der Titandioxidplättchen mit weiterem Titandioxid nach Trocknung auch in einem Wirbelbettreaktor durch Gasphasenbeschichtung erfolgen, wobei z.B. die in EP 0 045 8511 und EP 0 106 235 zur Herstellung von Perlglanzpigmenten vorgeschlagenen Verfahren entsprechend angewendet werden können.

Das erfindungsgemäße Pigment kann noch zusätzlich mit schwerlöslichen, fest haftenden anorganischen oder organischen Farbmitteln beschichtet werden. Bevorzugt werden Farblacke und insbesondere Aluminiumfarblacke verwendet. Dazu wird eine Aluminiumhydroxidschicht aufgefällt, die in einem zweiten Schritt mit einem Farblack verlackt wird. Das Verfahren ist in DE 24 29 762 und DE 29 28 287 näher beschrieben.

Bevorzugt ist auch eine zusätzliche Beschichtung mit Komplexsalzpigmenten, insbesondere Cyanoferratkomplexen, wie zum Beispiel Berliner Blau und Turnbulls Blau, wie sie in EP 0 141 173 und DE 23 13 332 beschrieben ist.

Das erfindungsgemäße Pigment kann auch mit organischen Farbstoffen und insbesondere mit Phthalocyanin- oder Metallphthalocyanin- und/oder Indanthrenfarbstoffen nach DE 40 09 567 beschichtet werden. Dazu wird eine Suspension des Pigmentes in einer Lösung des Farbstoffes hergestellt und diese dann mit einem Lösungsmittel zusammengebracht, in welchem der Farbstoff schwer löslich oder unlöslich ist.

Weiterhin können auch Metallchalkogenide bzw. Metalchalkogenidhydrate und Ruß für eine zusätzliche Beschichtung eingesetzt werden.

Es ist weiterhin möglich, das Pigment einer Nachbeschichtung oder Nachbehandlung zu unterziehen, die die Licht-, Wetter- und chemische Stabilität weiter erhöht, oder die Handhabung des Pigments, insbesondere die Einarbeitung in unterschiedliche Medien, erleichtert. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage.

Die zusätzlich aufgebrachten Stoffe machen nur etwa 0,5 bis 5 Gew.-%, vorzugsweise etwa 1 bis 3 Gew.-%, des gesamten Pigmentes aus.

Das Pigment kann auf übliche Weise zur Pigmentierung von Lebensmitteln, Lacken, Druckfarben, Kunststoffen, Kosmetika und Glasuren für Keramiken und Gläser verwendet werden. Es kann aber auch für den UV-Schutz in kosmetischen Formulierungen oder für technische Anwendungen eingesetzt werden, da es im UV-Bereich unterhalb von 330 nm praktisch vollständig die ultraviolette Strahlung abschirmt. Damit ist das Pigment im Handel befindlichen Lichtschutzfiltern deutlich überlegen.

Die Konzentration des Pigmentes in diesen Formulierungen beträgt 0,1 Gew.-% bis 30 Gew.-%, vorzugsweise 1 bis 10 Gew.-%.

Das erfindungsgemäße Pigment wurde bezüglich seiner abschirmenden Wirkung gegenüber der UV-Strahlung mit Luxelen Silk D (Hersteller: Presperse, Inc.), einem kommerziell verfügbaren Sonnenschutzmittel verglichen. Hierfür wurde das Pigment (Schichtdicke: 130 nm) und das Vergleichsprodukt in einen NC-Lack eingearbeitet (Pigmentkonzentration 1,7 %) und auf einer Glasplatte mit Hilfe einer Rakel ausgestrichen. Nach dem Trocknen wurden die Lackfilme von der Unterlage abgelöst und mit einem Meßgerät PE Lambda 19 mit eingebauter Integrationskugel (vergrößerte Specacarol-Blenden, 30 nm) bezüglich Transmission, Reflexion und Absorption im Bereich von 200 bis 2500 nm vermessen. Die Transmission des Lackfilmes mit dem erfindungsgemäßen Pigment beträgt unterhalb von 330 nm 0 %, bei 350 nm etwa 5 %, bei 380 nm 35 % und bei 400 nm 40 %. Die Durchlässigkeit für die UV-Strahlung unterhalb von 350 nm ist damit deutlich geringer als beim Vergleichsprodukt.

Das erfindungsgemäße Pigment stellt bezüglich der Dicke den Idealzustand dar, der bei Perlglanzpigmenten maximal erreichbar ist.

Die Plättchendicke entspricht im vorliegenden Fall der erforderlichen Schichtdicke für die optisch funktionelle TiO₂-Schicht, wohingegen bei konventionellen Perlglanzpigmenten die Plättchendicke um den Faktor 25 größer sein kann, da zur funktionellen Schicht noch die Schichtdicke des Substrates, beispielsweise Glimmer, kommt.

Bezüglich der technischen Anwendungen ergeben sich hieraus intrinsische Vorteile, die von keinem anderen konventionellen Perglanzpigment erreicht werden.

Beispielsweise können Lackschichten dünner ausgeführt und die notwendige Pigmentmenge reduziert werden, weil aufgrund des Fehlens des "Füllstoffs" Trägermaterial die Pigmente optisch effizienter sind.

Die nachfolgenden Beispiele sollen die Erfindung erläutern, ohne sie zu begrenzen.

### Beispiel 1

Ein umlaufendes Band aus Polyethylenterephthalat (Breite: 0,3 m, Geschwindigkeit: 20 m/min) wird über eine Auftragswalze im Gegenlauf mit einer 20%iger Titantetrachloridlösung beschichtet. Die Beschichtungslösung enthält 0,3 Gew.-% Tensid (DISPERSE-AYD W-28, Hersteller: DANIEL PRODUCTS COMPANY). Der auf dem Band befindliche wäßrige Film wird in einer Trocknungsstrecke durch Beaufschlagung mit Heißluft von 70 °C getrocknet und die gebildete Schicht in einem mit vollentsalztem Wasser gefüllten Ablösebecken vom Band abgelöst. Die Titandioxidpartikel werden filtriert und mit vollentsalztem Wasser gewaschen. Die silbrig glänzenden Plättchen haben eine Schichtdicke von 100 ± 10 nm. Zur Beschichtung mit weiterem Titandioxid werden sie in vollentsalztem Wasser redispergiert.

2 I der Dispersion von TiO₂-Plättchen (Trockensubstanzgehalt: 15 g TiO₂) aus Beispiel 1 werden auf 75 °C erhitzt und mit verdünnter Salzsäure auf einen pH-Wert von 2,2 eingestellt.

Eine 40%ige wäßrige Titantetrachloridlösung wird nun mit 3 ml/min zudosiert und der pH-Wert weiterhin mit 32%iger NaOH konstant auf 2,2 gehalten.

Die TiCl₄-Zugabe wird fortgesetzt bis die gewünschte Interferenzfarbe erster oder höherer Ordnung erreicht ist. Das erhaltene Pigment wird abfiltriert, mit VE-Wasser salzfrei gewaschen, getrocknet und bei 750 °C geglüht. Die koloristischen Eigenschaften ändern sich in Abhängigkeit von der Glühtemperatur, wobei die Anatas-Rutil-Umwandlung, die bei ca. 600 °C beginnt und bei 750 °C abgeschlossen ist, eine wichtige Rolle spielt.

### Beispiel 2

### Sonnenschutzcreme

### Bestandteile

| | | |
|---|---|---|
| Komponente A | Flüssiges Paraffin | 20,0 % |
| | Cetylalkohol | 1,5 % |
| | Bienenwachs | 6,0 % |
| | Stearinsäure | 20,0 % |
| | POE (5,5) Cetylether | 1,5 % |
| | Sorbitanmonostearat | 2,5 % |
| | | |
| Komponente B | 10%ige NaOH | 1,0 % |
| | Destilliertes Wasser | 36,5 % |
| | | |
| Komponente C | Glycerin | 6,0 % |
| | TiO₂-Pigment | 5,0 % |

### Herstellung:

Das Pigment wird in Glycerin dispergiert. Die Komponenten A und B werden getrennt auf 75 °C erhitzt und mit Hilfe eines Hochgeschwindigkeitsrührwerks ineinander eingeliert. Schließlich wird die Komponente C in der Emulsion von A und B bei 50 °C emulgiert.

## Patentansprüche

1. Plättchenförmiges Titandioxidpigment mit einer Schichtdicke von kleiner als 500 nm und einer Schichtdickentoleranz von weniger als 10%, erhältlich durch Verfestigung einer wäßrigen Lösung einer thermisch hydrolysierbaren Titanverbindung auf einem endlosen Band, Ablösung der entstandenen Schicht, Beschichtung der erhaltenen Titandioxidplättchen ohne Zwischentrocknung im Naßverfahren mit weiterem Titandioxid, Abtrennung, Trocknung und Kalzination des erhaltenen Materials.

2. Titandioxidpigment nach Anspruch 1, **dadurch gekennzeichnet, daß** als thermisch hydrolysierbare Titanverbindung eine wäßrige Titantetrachloridlösung eingesetzt wird.

3. Verfahren zur Herstellung des Titandioxidpigmentes nach den Ansprüchen 1 und 2, **dadurch gekennzeichnet, daß**
- eine wäßrige Lösung einer thermisch hydrolysierbaren Titanverbindung als dünner Film auf ein endloses Band aufgebracht wird,
- der flüssige Film durch Trocknung verfestigt wird und dabei das Titandioxid durch eine chemische Reaktion aus der Lösung entwickelt wird,
- die entstandene Schicht anschließend vom Band abgelöst und gewaschen wird,
- die erhaltenen Titandioxidplättchen ohne Zwischentrocknung in Wasser suspendiert und mit weiterem Titandioxid beschichtet werden,
- die Titandioxidplättchen aus der wäßrigen Suspension abgetrennt, getrocknet und kalziniert werden.

4. Verfahren nach Anspruch 3, **dadurch gekennzeichnet, daß** die wäßrige Lösung einer thermisch hydrolysierbaren Titanverbindung eine wäßrige Titantetrachloridlösung ist.

5. Verfahren nach den Ansprüchen 3 und 4, **dadurch gekennzeichnet, daß** das weitere Titandioxid in einem Wirbelbettreaktor durch CVD auf die getrockneten Titandioxidplättchen aufgebracht wird.

6. Verwendung der Pigmente nach den Ansprüchen 1 und 2 zur Pigmentierung von Lacken, Druckfarben, Kunststoffen, Kosmetika und Glasuren für Keramiken und Gläser und für Nahrungsmittel sowie als Sonnenschutzmittel.

7. Verwendung nach Anspruch 6, **dadurch gekennzeichnet, daß** die Pigmente als Mischungen mit handelsüblichen Pigmenten eingesetzt werden.

8. Lacke, Druckfarben, Kunststoffe, Kosmetika, Keramiken und Gläser, welche mit einem Pigment nach den Ansprüchen 1 und 2 pigmentiert sind.

## Claims

1. Plateletlike titanium dioxide pigment having a layer thickness of less than 500 nm and a layer-thickness tolerance of less than 10%, obtainable by solidifying an aqueous solution of a thermally hydrolysable titanium compound on a continuous belt, detaching the resulting layer, coating the resulting titanium dioxide platelets, without drying in between, with further titanium dioxide by a wet method, separating out, drying and calcining the material obtained.

2. Titanium dioxide pigment according to Claim 1, **characterized in that** the thermally hydrolysable titanium compound employed is an aqueous titanium tetrachloride solution.

3. Process for the preparation of the titanium dioxide pigment according to Claims 1 and 2, **characterized in that**
- an aqueous solution of a thermally hydrolysable titanium compound is applied as a thin film to a continuous belt
- the liquid film is solidified by drying, during the course of which the titanium dioxide is developed from the solution by means of a chemical reaction,
- the resulting layer is subsequently detached from the belt and washed,
- the titanium dioxide platelets obtained, without drying in between, are suspended in water and coated with further titanium dioxide,
- the titanium dioxide platelets are separated out from the aqueous suspension, dried and calcined.

4. Process according to Claim 3, **characterized in that** the aqueous solution of a thermally hydrolysable titanium compound is an aqueous titanium tetrachloride solution.

5. Process according to Claims 3 and 4, **characterized in that** the further titanium dioxide is applied to the dried titanium dioxide platelets in a fluidized-bed reactor by CVD.

6. Use of the pigments according to Claims 1 and 2 for pigmenting paints, printing inks, plastics, cosmetics and glazes for ceramics and glass, and for foodstuffs and as sun protection agents.

7. Use according to Claim 6, **characterized in that** the pigments are employed as mixtures with commercially available pigments.

8. Paints, printing inks, plastics, cosmetics, ceramics and glass which are pigmented with a pigment according to Claims 1 and 2.

## Revendications

1. Pigment de dioxyde de titane sous forme de paillettes, ayant une épaisseur de couche inférieure à 500 nm et une tolérance d'épaisseur de couche de moins de 10 %, pouvant être obtenu par solidification, sur une bande continue, d'une solution aqueuse d'un composé contenant du titane, hydrolysable à la chaleur, détachement de la couche formée, enrobage avec une nouvelle quantité de dioxyde de titane des paillettes de dioxyde de titane obtenues, dans le procédé par voie humide, sans séchage intermédiaire, séparation, séchage et calcination du produit obtenu.

2. Pigment de dioxyde de titane selon la revendication 1, **caractérisé en ce qu'**on utilise comme composé contenant du titane, hydrolysable à la chaleur, une solution aqueuse de tétrachlorure de titane.

3. Procédé pour la préparation du pigment de dioxyde de titane selon les revendications 1 et 2, **caractérisé en ce que**
- on applique sur une bande continue une solution aqueuse d'un composé contenant du titane, hydrolysable à la chaleur, sous forme d'une mince pellicule,
- la mince pellicule est solidifiée par séchage et pendant ce séchage le dioxyde de titane se développe par une réaction chimique à partir de la solution,
- la couche formée est ensuite détachée de la bande et lavée,
- les paillettes de dioxyde de titane obtenues sont mises en suspension dans de l'eau, sans séchage intermédiaire, et enrobées avec une nouvelle quantité de dioxyde de titane,
- les paillettes de dioxyde de titane sont séparées de la suspension aqueuse, séchées et calcinées.

4. Procédé selon la revendication 3, **caractérisé en ce que** la solution aqueuse d'un composé contenant du titane, hydrolysable à la chaleur, est une solution aqueuse de tétrachlorure de titane.

5. Procédé selon les revendications 3 et 4, **caractérisé en ce que** la nouvelle quantité de dioxyde de titane est appliquée sur les paillettes de dioxyde de titane séchées, par CVD (dépôt chimique en phase vapeur) dans un réacteur à lit fluidisé.

6. Utilisation des pigments selon les revendications 1 et 2, pour la pigmentation de peintures, d'encres d'imprimerie, de matières plastiques, de cosmétiques et de glaçures pour céramiques et verres, et pour des produits alimentaires ainsi que des produits anti-solaires.

7. Utilisation selon la revendication 6, **caractérisée en ce que** les pigments sont utilisés sous forme de mélanges avec des pigments du commerce.

8. Peintures, encres d'imprimerie, matières plastiques, cosmétiques, céramiques et verres, pigmentés avec un pigment selon les revendications 1 et 2.
